# EUROPEAN PATENT APPLICATION

(11) **EP 4 497 773 A1**
(43) Date of publication of application: **29.01.2025**
(21) Application number: 23774592.2
(22) Date of filing: 10.03.2023
(51) Int. Cl.: C08J 3/12, A61K 8/02, A61K 8/73, A61K 8/85, A61Q 1/00, C08J 3/18, C08L 101/16

(54) **BIODEGRADABLE FLATTENED PARTICLES, COSMETIC COMPOSITION AND METHOD FOR PRODUCING BIODEGRADABLE FLATTENED PARTICLES**

(30) Priority: 23.03.2022 JP 2022047148
(71) Applicant: Daicel Corporation, Osaka-shi, Osaka 530-0011 (JP)
(72) Inventor: KOBAYASHI, Keiko, Tokyo 108-8230 (JP); SAKAMOTO, Yuta, Tokyo 108-8230 (JP); OMURA, Masaya, Tokyo 108-8230 (JP)
(74) Representative: Grünecker Patent- und Rechtsanwälte PartG mbB
(86) International application number: PCT/JP2023/009305
(87) International publication number: WO 2023/181993

(57) **Abstract**

[Problem] To provide a biodegradable flattened particle having good tactile sensation and improved soft focus properties, and a production method thereof.

[Solution] A biodegradable flattened particle contains a biodegradable polymer as a major ingredient. This biodegradable flattened particle has an oblateness L/T, which is a ratio of an average long diameter L to an average thickness T, of 2.0 or more and a surface smoothness of 80% or more. A method for producing this biodegradable flattened particle includes: mixing a biodegradable polymer, a plasticizer, and a water-soluble polymer to prepare a mixture; melt-kneading this mixture at a temperature of 200°C or higher and 280°C or lower to prepare a kneaded mixture; pressurizing this kneaded mixture at a temperature lower than the melting point of this water-soluble polymer; and removing the water-soluble polymer from this pressurized kneaded mixture.

## Description

### [Technical Field]

The present disclosure relates to a flattened particle and a production method thereof, and a cosmetic composition containing the flattened particle.

### [Background Art]

Conventionally, various fine particles have been formulated for the purpose of enhancing the spreadability of cosmetics, giving a change to tactile sensation, imparting a wrinkle blurring effect, or enhancing the slipperiness of a foundation or the like. Depending on the shape or properties of fine particles, a light-scattering (soft focus) effect can be elicited. In particular, when fine particles with a flattened shape are used in a foundation or the like, effects of improving optical functions, such as preventing dullness of a cosmetic film and imparting transparency to the cosmetic film, or concealing wrinkles and unevenness of the skin to make the skin more uniform, can be elicited.

As the materials for the flattened particles formulated in such cosmetics, inorganic substances, such as mica flakes, sericite, and talc, are normally used. The flattened particles composed of these inorganic substances may be used after being surface-treated, but may have a problem of poor slipperiness and tactile sensation on the skin. Meanwhile, fine particles composed of synthetic polymers such as polyamide, polymethyl methacrylate, polystyrene, polypropylene, and polyethylene exhibit better tactile sensation than inorganic fine particles. However, in recent years, as an alternative to these synthetic polymers, fine particles composed of biodegradable materials that have the necessary properties and are environmentally friendly have been demanded due to the problem of marine pollution caused by microplastics.

As a non-synthetic polymer, an application of fine particles composed of naturally occurring cellulose has been studied. For example, Japanese Patent Application Publication No. 2011-127124 (PTL 1) discloses flattened cellulose particles with an average particle diameter of 1 to 100 µm, an average thickness of 0.01 to 5 um, and an oblateness of 20 to 200. These flattened cellulose particles are manufactured by mechanically grinding a mixture containing a cellulose-based substance.

### [Citation List]

### [Patent Literature]

[PTL 1] Japanese Patent Application Publication No. 2011-127124

### [Summary of Invention]

### [Technical Problem]

The flattened cellulose particle disclosed in PTL 1 is mechanically ground. By the mechanical grinding, the surface smoothness of the resulting particle is impaired. If these flattened cellulose particles are used in a foundation or the like, for example, sufficient tactile sensation may not be obtained due to the surface shape thereof.

Flattened particles that are made from a biodegradable polymer and elicit both excellent optical effects and tactile improvement effects have not yet been proposed. An object of the present disclosure is to provide a biodegradable flattened particle with good tactile sensation and improved soft focus properties, and a production method thereof.

### [Solution to Problem]

A biodegradable flattened particle of the present disclosure contains a biodegradable polymer as a major ingredient. This biodegradable flattened particle has an oblateness D/T, which is a ratio of an average long diameter L (um) to an average thickness T (µm), of 2.0 or more and a surface smoothness of 80% or more.

This biodegradable flattened particle may have an aspect ratio L/S, which is a ratio of an average long diameter L (um) to an average short diameter S (µm), of 2.0 or more.

The biodegradable polymer is preferably selected from the group consisting of a polysaccharide, a polysaccharide ester, and an aliphatic polyester. The polysaccharide(s) may be one or two types of polysaccharides selected from cellulose and starch. The polysaccharide ester may have a total substitution degree of over 0 and 3.0 or less. The polysaccharide ester may be a cellulose acylate with a C2-10 acyl group.

The aliphatic polyester may be a polyhydroxyalkanoic acid or a polymer of an aliphatic dicarboxylic acid and an aliphatic diol. The aliphatic polyester may be one or two or more types of aliphatic polyesters selected from the group consisting of polycaprolactone, polyhydroxybutyric acid, and polylactic acid.

A cosmetic composition of the present disclosure contains any biodegradable flattened particle described above.

A method for producing a biodegradable flattened particle of the present disclosure includes:
(1) mixing a biodegradable polymer, a plasticizer, and a water-soluble polymer to prepare a mixture;
(2) melt-kneading the mixture at a temperature of 200°C or higher and 280°C or lower to prepare a kneaded mixture;
(3) pressurizing the kneaded mixture at a temperature lower than the melting point of the water-soluble polymer;
   and
(4) removing the water-soluble polymer from the pressurized kneaded mixture.

In this production method, the kneaded mixture may be pressurized at a temperature of 150°C or higher and 200°C or lower. In this production method, the kneaded mixture may be pressurized at a pressure of 500 MPa or higher.

### [Advantageous Effects of Invention]

According to the present disclosure, a biodegradable flattened particle with good tactile sensation and improved optical properties, such as soft focus properties, and a cosmetic composition containing this biodegradable flattened particle can be provided.

### [Brief Description of Drawings]

[Fig. 1]

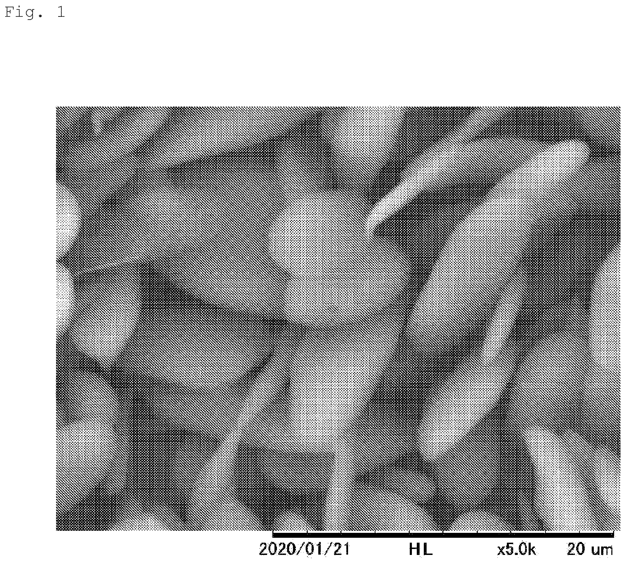

   Fig. 1 is a scanning electron micrograph (SEM) (magnification of 5000×) of flattened particles in Example A-1.
[Fig. 2]

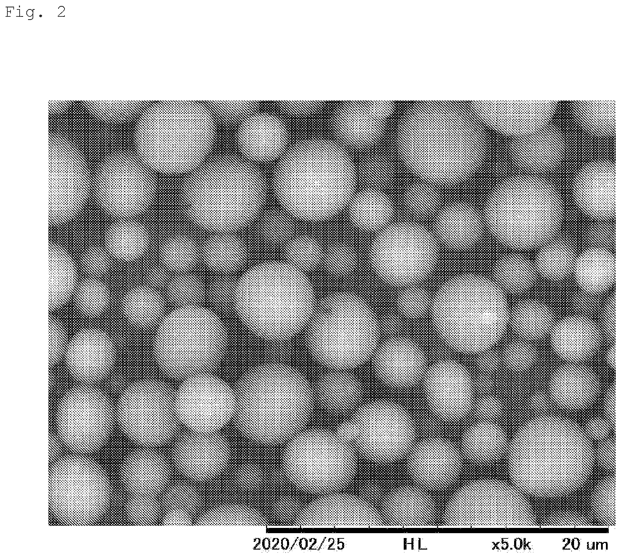

   Fig. 2 is a scanning electron micrograph (SEM) (magnification of 5000×) of particles in Comparative Example A-1.

### [Description of Embodiments]

Hereinafter, the present disclosure will be described in detail on the basis of preferred embodiments. Each constitution and combination thereof or the like in each embodiment are illustrative, and additions, omissions, substitutions, and other modifications of constitutions may be made as appropriate without departing from the spirit of this disclosure. The present disclosure is not limited by embodiments and only limited by the scope of claims. Each embodiment disclosed in the present description may be combined with any other features disclosed in the present description.

In the description of the present application, "X to Y" indicating a range means "X or greater and Y or less", "ppm" means "ppm by weight", and all test temperatures are room temperature (20°C ± 5°C) unless otherwise noted.

### [Flattened Particle]

The biodegradable flattened particle of the present disclosure (which may be referred to as a "flattened particle" hereinafter) contains a biodegradable polymer as a major ingredient. This flattened particle has an oblateness L/T, which is a ratio of an average long diameter L (um) to an average thickness T (µm), of 2.0 or more and a surface smoothness of 80% or more. Here, a "major ingredient" means that the most abundant ingredient among the constituent ingredients of particles is a biodegradable polymer and means that the content thereof is at least 50% by weight. The term "biodegradable polymer" means a polymer that is degraded in soil or seawater, or a living body. In the present disclosure, the term "polymer" is defined as a compound composed of one or two or more types of constituent units repeatedly bonded together. As long as a polymer shows a certain level of biodegradability, the polymer may be a synthetic polymer or a naturally derived polymer.

The flattened particle of the present disclosure is composed of a biodegradable material and has excellent soft focus properties and good tactile sensation due to its shape. The flattened particle of the present disclosure may be formulated in various cosmetic compositions. Formulating this flattened particle can provide a high-quality cosmetic composition with less environmental load.

The flattened particle of the present disclosure has an oblateness L/T, a ratio of an average long diameter L (µm) to an average thickness T (µm), of 2.0 or more. Here, the term "long diameter" of the flattened particle is defined as a maximum diameter in a projected plane obtained by projecting a particle on a two-dimensional plane such that the area of this particle should be the largest. In other words, the length of the long side of the smallest rectangle circumscribed by the projected plane of this particle is the "long diameter" of the present disclosure. The term "thickness" is defined as the length in the direction perpendicular to a projected plane obtained by projecting the particle on a two-dimensional plane such that the area of the particle should be the largest.

This flattened particle with an oblateness of 2.0 or more exhibits high optical effects due to the particle shape. In particular, this flattened particle can provide a cosmetic composition with improved soft focus properties. From the viewpoint of improvement of optical effects, the oblateness of the flattened particle may be 3.0 or more, may be 5.0 or more, or may be 7.5 or more. From the viewpoint of minimal impact on tactile sensation and ease of formulation in a cosmetic composition, the oblateness of the flattened particle may be 30 or less, may be 25 or less, or may be 20 or less. In the present description, the term "tactile sensation" is a concept that includes not only the tactile sensation of directly touching the flattened particle, but also, for example, the skin texture and tactile sensation when the flattened particle is formulated in a cosmetic composition.

In the present disclosure, the oblateness of the flattened particle can be determined by taking a scanning electron micrograph of particles, measuring the long diameter and the thickness of a plurality of particles, and calculating a ratio L/T of an average long diameter L (um) to an average thickness T (um). Details of the measurement method are described later in Examples.

The surface smoothness of the flattened particle of the present disclosure is 80% or more, and preferably 85% or more, more preferably 90% or more, and further preferably 95% or more, and the upper limit is 100%. If the surface smoothness is less than 80%, the desired tactile sensation may not be obtained in some cases. From the viewpoint that it is easy to obtain good tactile sensation, the surface smoothness of the flattened particle may be 80% to 100%, may be 85% to 100%, may be 90% to 100%, or may be 95% to 100%.

The surface smoothness of the flattened particle can be obtained based on the area of recesses by taking a scanning electron micrograph of particles and observing the projections and recesses on the particle surface. Details of a method of measuring surface smoothness are described later in Examples.

The shape of the flattened particle of the present disclosure may be any shape having the oblateness and surface smoothness described above and is not particularly limited. However, from the viewpoint that it is easy to obtain the desired tactile sensation and optical properties, a shape with a contour formed by rounded curves and with few or almost no sharp edges is preferred. Examples of the shapes of the flattened particle include flat ellipsoids and substantially ellipse ellipsoids. In other words, the flattened particle of the present disclosure has two flat surfaces with contour lines formed by curves and side surfaces located between these two flat surfaces. These side surfaces are substantially smooth curved surfaces that continue smoothly from the flat surfaces, and the flattened particle of the present disclosure differs from conventional flat plate particles and scaly particles in this respect.

From the viewpoint that it is easy to obtain optical effects, the flattened particle of the present disclosure may have an aspect ratio L/S, which is a ratio of an average long diameter L (um) with respect to an average short diameter S (µm), of 2.0 or more, 2.2 or more, or 2.5 or more. From the viewpoint that tactile sensation is not inhibited, this aspect ratio may be 10 or less. Here, the term "long diameter" of the flattened particle is as described above, and the term "short diameter" is defined as the length of the short side of the smallest rectangle circumscribed by the projected plane obtained by projecting a particle on a two-dimensional plane such that the area of this particle should be the largest.

In the present disclosure, the aspect ratio of the flattened particle can be determined by taking a scanning electron micrograph of particles, measuring the long diameter and short diameter of a plurality of particles, and calculating a ratio L/S of an average long diameter L (um) to an average short diameter S (um). Details of the measurement method are described later in Examples.

The average long diameter of the flattened particle of the present disclosure may be 0.5 um or longer, may be 1.0 um or longer, or may be 2.0 um or longer, and may be 100 um or shorter, may be 80 um or shorter, or may be 60 um or shorter. If the average long diameter exceeds 100 µm, the tactile sensation may be poor in some cases. Flattened particles with an average particle diameter of less than 0.5 µm are difficult to manufacture.

The average short diameter of the flattened particle of the present disclosure may be 0.1 µm or longer, may be 0.5 um or longer, or may be 1.0 µm or longer, and may be 50 µm or shorter, may be 40 µm or shorter, or may be 30 µm or shorter. If the average short diameter exceeds 50 um, the tactile sensation may be poor in some cases. Flattened particles with an average short diameter of less than 0.1 um are difficult to manufacture.

The average thickness of the flattened particle of the present disclosure may be 0.05 µm or thicker, may be 0.1 µm or thicker, or may be 0.2 µm or thicker, and may be 5.0 µm or thinner, may be 4.0 µm or thinner, or may be 3.0 µm or thinner. If the average thickness exceeds 3.0 µm, the effect due to the flat shape is less likely to be obtained. Flattened particles with an average thickness of less than 0.05 µm are difficult to manufacture.

The main ingredient of the flattened particle of the present disclosure may be a biodegradable polymer selected from the group consisting of polysaccharides, polysaccharide esters, and aliphatic polyesters. In the range that the effect of the present disclosure can be obtained, the flattened particle may further contain a biodegradable polymer, such as an aliphatic polyol, an aliphatic polycarbonate, a polyacid anhydride, or the like.

The term "polysaccharides" means polymer compounds in which monosaccharides are bonded by glycoside bonds. As long as the effect of the present disclosure can be obtained, polysaccharides may be a polymer of α-glucose or a polymer of β-glucose. Examples of polysaccharides may include cellulose, hemicellulose, pullulan, amylose, agarose, chitin, chitosan, carrageenan, pectin, dextrin, starch, collagen, mannan, arabinogalactan, glycogen, inulin, hyaluronic acid, and modified substances of these. Two or more types of polysaccharides may be used together. The polysaccharides are preferably one or two types of polysaccharides selected from cellulose and starch.

A polysaccharide ester is a carboxylic acid ester of polysaccharides described above and is defined as a compound in which some of the hydroxy groups in a molecular chain are replaced with acyl groups. The polysaccharide ester is preferably one or two types of esters of polysaccharides selected from cellulose and starch. Two or more types of polysaccharide esters may be used together. Carboxylic acid esters of other polysaccharides that are not explicitly described in the present description may be used within the range that the effect of the present disclosure can be obtained.

The total substitution degree of a polysaccharide ester used in the flattened particle of the present disclosure is selected, as appropriate, within the range of over 0 and 3.0 or less according to the type of polysaccharides and the type of substituents. The total substitution degree of the polysaccharide ester may be 0.3 or more, may be 0.5 or more, may be 0.7 or more, may be 1.0 or more, or may be 1.2 or more, and may be 2.95 or less, may be 2.80 or less, or may be 2.65 or less. The total substitution degree of the polysaccharide ester may be determined by a known method using ¹³C-NMR or ¹H-NMR.

From the viewpoint of ease of availability and excellent biodegradability, preferred polysaccharide esters are cellulose esters, and a cellulose acylate with an acyl group containing two or more carbon atoms is more preferred. The number of carbons in an acyl group in a cellulose acylate may be 3 or more, or may be 4 or more, and may be 10 or less, or may be 8 or less. The cellulose acylate may have two or more types of acyl groups as substituents. In the present disclosure, two or more types of cellulose acylates of different degrees of substitution and different numbers of carbons in an acyl group may be used together as a biodegradable polymer.

Specific examples of cellulose acylates in the present disclosure may include cellulose acetate, cellulose propionate, cellulose butyrate, cellulose acetate propionate, cellulose acetate butyrate, and the like. From the viewpoint of biodegradability and ease of availability, cellulose acetate, cellulose acetate propionate, and cellulose acetate butyrate are preferred.

From the viewpoint that good biodegradability can be obtained, the total substitution degree of a cellulose acylate may be 3.0 or less, may be 2.95 or less, may be 2.80 or less, or may be 2.65 or less. From the viewpoint that it is easy to obtain the desired shape, the total substitution degree of a cellulose acylate may exceed 0, may be 0.3 or more, may be 0.5 or more, may be 0.7 or more, may be 1.0 or more, may be 1.20 or more, may be 1.50 or more, or may be 2.10 or more.

The degree of substitution of a cellulose acylate may be measured by the following method. For example, the degree of substitution of a cellulose acylate may be measured by an NMR method in accordance with the method proposed by Tezuka (Tezuka, Carbonydr. Res. 273, 83 (1995)). That is, free hydroxy groups in a cellulose acylate are acylated with a carboxylic acid anhydride in pyridine. The type of carboxylic anhydrides used herein should be selected according to the analysis purpose, and, for example, acetic anhydride is preferred when the degree of propionyl substitution of a cellulose propionate is analyzed. An obtained sample is dissolved in deuterated chloroform, and ¹³C-NMR spectrum is measured. When the degree of propionyl substitution is analyzed after treating a cellulose acylate that has propionyl groups or a cellulose acylate that does not have propionyl groups with propionic anhydride, the signals of carbonyl carbons in propionyl groups appear in the same order in the region from 172 ppm to 174 ppm in the order of positions 2, 3, and 6 from the higher field. The total substitution degree of a cellulose acylate treated with a carboxylic anhydride in accordance with the method of Tezuka or other equivalent methods is 3.0. Thus, if the sum of the area of the carbonyl carbon signals of the acyl group originally present in a cellulose acylate and the carbonyl signals of the acyl group introduced by the carboxylic anhydride treatment is normalized to 3.0, and the abundance ratio (that is, the area ratio of each signal) of each acyl group at a corresponding position is determined, the determined values are regarded as acyl substitution degrees of positions 2, 3, and 6, respectively, in a glucose ring of a cellulose ester. It goes without saying that the substituents including acyl groups that can be analyzed by this method are only substituents that do not correspond to carboxylic anhydride used in treatment for analysis purposes. The degree of substitution may be analyzed not only by ¹³C-NMR, but also by ¹H-NMR.

As long as the effect of the present disclosure can be obtained, the weight average molecular weight of the cellulose acylate is not particularly limited. From the viewpoint that it is easy to obtain the desired shape, the weight average molecular weight of the cellulose acylate is preferably 10,000 or more, more preferably 20,000 or more, and further preferably 30,000 or more. From the viewpoint that biodegradability is high and it is easy to obtain the desired flat shape, the weight average molecular weight of the cellulose acylate is preferably 500,000 or less, more preferably 400,000 or less, and further preferably 300,000 or less. The weight average molecular weight of a cellulose ester of a cellulose acylate is measured similarly for measuring the aliphatic polyester described above.

The type of aliphatic polyesters is not particularly limited. For example, from the viewpoint of polymer structures, polyhydroxyalkanoic acids with constituent units of polycondensed hydroxyalkanoic acid as repeating units and polymers with constituent units of dehydrated and condensed aliphatic dicarboxylic acid and aliphatic diol as repeating units may be mentioned.

Examples of polyhydroxyalkanoic acids may include polyglycol acid, polylactic acid, poly(β-hydroxybutyrate), poly(β-hydroxyvalerate), poly(lactic-co-glycolic acid), poly(β-hydroxybutyrate-co-β-hydroxyvalerate), poly(β-propiolactone), poly(ε-caprolactone), and the like. Examples of polymers of an aliphatic dicarboxylic acid and an aliphatic diol may include poly(ethylene succinate), poly(butylene succinate), poly(butylene succinate-co-butylene adipate), and the like. Two or more types of them may be used together.

From the viewpoint of ease of availability and excellent biodegradability, preferable aliphatic polyester(s) is/are one or two or more types of aliphatic polyesters selected from the group consisting of polycaprolactone, polyhydroxybutyric acid, and polylactic acid. Other aliphatic polyesters that are not explicitly described in the present description may be used within the range that the effect of the present disclosure can be obtained.

From the viewpoint that it is easy to obtain the desired shape, the weight average molecular weight of the aliphatic polyester is preferably 10,000 or more, more preferably 20,000 or more, and further preferably 50,000 or more. From the viewpoint of excellent biodegradability, the weight average molecular weight of the aliphatic polyester is preferably 5,000,000 or less, more preferably 1,000,000 or less, still more preferably 500,000 or less, and particularly preferably 250,000 or less.

The weight average molecular weight of the aliphatic polyester may be determined by a size exclusion chromatography (GPC) measurement using the following apparatus and condition (GPC-light scattering method).
Apparatus: Shodex GPC "SYSTEM-21H"
Solvent: acetone
Column: two GMHxl columns (Tosoh), a guard column (TSK-gel guard column HXL-H, manufactured by Tosoh Corporation)
Flow rate: 0.8 ml/min
Temperature: 29°C
Sample concentration: 0.25% (wt/vol)
Injection amount: 100 ul
Detection: MALLS (multi-angle light scattering detector)
("DAWN-EOS", manufactured by Wyatt Technology Corporation)
Standard substance for MALLS correction: PMMA (molecular weight: 27600)

As another embodiment of the present disclosure, the flattened particle may contain a plasticizer. The plasticizer in the present disclosure refers to a compound that can increase the plasticity of the biodegradable polymer described above. The type of plasticizers are not particularly limited, and examples of plasticizers may include polyhydric carboxylic acid esters, for example, adipic acid-based plasticizers, including adipic acid esters, such as dimethyl adipate, dibutyl adipate, diisostearyl adipate, diisodecyl adipate, diisononyl adipate, diisobutyl adipate, diisopropyl adipate, diethylhexyl adipate, dioctyl adipate, dioctyldodecyl adipate, dicapryl adipate, dihexyldecyl adipate, di(ethylene glycol monoalkyl ether) adipate, di(diethylene glycol monomethyl ether) adipate, di(triethylene glycol monomethyl ether) adipate, di(tetraethylene glycol monomethyl ether) adipate, di(pentaethylene glycol monomethyl ether) adipate, di(hexaethylene glycol monomethyl ether) adipate, di(propylene glycol monoalkyl ether) adipate, di(dipropylene glycol monomethyl ether) adipate, di(tripropylene glycol monomethyl ether) adipate, di(tetrapropylene glycol monomethyl ether) adipate, di(pentapropylene glycol monomethyl ether) adipate, di(hexapropylene glycol monomethyl ether) adipate, di(polyethylene glycol monoalkyl ether) adipate, di(polypropylene glycol monoalkyl ether) adipate, diphenyl adipate, dinaphthyl adipate, and dibenzyl adipate; citric acid-based plasticizers, including citric acid esters, such as acetyltriethyl citrate, acetyltributyl citrate, isodecyl citrate, isopropyl citrate, triethyl citrate, triethylhexyl citrate, and tributyl citrate; glutaric acid-based plasticizers, including glutaric acid esters, such as diisobutyl glutarate, dioctyl glutarate, and dimethyl glutarate; succinic acid-based plasticizers, including succinic acid esters, such as diisobutyl succinate, diethyl succinate, diethylhexyl succinate, and dioctyl succinate; sebacic acid-based plasticizers, including sebacic acid esters, such as diisoamyl sebacate, diisooctyl sebacate, diisopropyl sebacate, diethyl sebacate, diethylhexyl sebacate, and dioctyl sebacate; and Phthalic acid-based plasticizers, including phthalic acid esters, such as ethyl phthalate, methyl phthalate, diaryl phthalate, diethyl phthalate, diethylhexyl phthalate, dioctyl phthalate, dibutyl phthalate, and dimethyl phthalate. These polyhydric carboxylic acid esters may be a mixed group polybasic acid ester.

Examples of plasticizers included in the flattened particle of the present disclosure may include glycerin-based plasticizers, including glycerin alkyl esters, such as triacetin, diacetin, and monoacetin; neopentyl glycol; phosphate-based plasticizers, including phosphoric acid esters, such as trioleyl phosphate, tristearyl phosphate, and tricetyl phosphate; bis(2-methoxyethyl) phthalate, dibutyl tartrate, ethyl o-benzoylbenzoate, ethyl phthalyl ethyl glycolate (EPEG), methyl phthalyl ethyl glycolate (MPEG), N-ethyl-toluenesulfonamide, o-cresyl p-toluenesulfonate, triethyl phosphate (TEP), triphenyl phosphate (TPP), tribrobionin, and the like. The flattened particle may contain one or two or more types of plasticizers.

From the viewpoint of high plasticizing effect on biodegradable polymers, a polyhydric carboxylic acid-based plasticizer or a glycerin-based plasticizer is preferred, and one or two or more types of plasticizers selected from a mixed group polybasic acid ester or a glycerin alkyl ester are more preferred. Examples of plasticizers for biodegradable polymers may include the trade name "DAIFATTY-10" and the like, manufactured by Daihachi Chemical Industry Co., Ltd., trade name "BIOCIZER", "Rikemal PL-004", "Poem G-002", and the like, manufactured by Riken Vitamin Co., Ltd., and trade name "Polycizer", "Monocizer", and the like, manufactured by DIC Corporation.

If the flattened particle of the present disclosure contains a plasticizer, the content of the plasticizer included in the flattened particle is not particularly limited. For example, the content of the plasticizer in the flattened particle may be over 0 parts by weight and 120 parts by weight or less, may be 2 parts by weight or more and 100 parts by weight or less, may be 10 parts by weight or more and 80 parts by weight or less, or may be 15 parts by weight or more and 50 parts by weight or less in relation to 100 parts by weight of the biodegradable polymer. The content of the plasticizer in the flattened particle can be determined by ¹H-NMR measurement.

As yet another embodiment of the present disclosure, a part or all of the surface of the flattened particle may be covered with inorganic powder. Inorganic powder existing on the particle surface provides, to the flattened particle, surface properties suitable for solvents and formulations used in cosmetic compositions. The flattened particle having inorganic powder on the surface thereof achieves high particle dispersibility in various solvents and formulations and improves the tactile sensation of the resulting cosmetic composition. The inorganic powder may be physically attached or chemically bonded to the flattened particle.

As long as the effect of the present disclosure can be obtained, the particle shape of inorganic powder is not particularly limited and may be, for example, any of spherical, plate-like, needle-like, granular, and irregular shapes. The average particle diameter of inorganic powder is preferably smaller than the average particle diameter of the flattened particle and, for example, may be 1/3 or less or may be 1/10 or less of the average particle diameter of the flattened particle. The average particle diameters of the inorganic powder and the flattened particle herein mean median sizes on a volume basis.

The type of inorganic powder is not particularly limited, and examples thereof may include titanium oxide, silicon oxide, aluminum oxide, zinc oxide, zirconium oxide, magnesium oxide, boron nitride, silicon nitride, barium sulfate, calcium sulfate, magnesium sulfate, calcium carbonate, magnesium carbonate, talc, mica, kaolin, sericite, mica, vermiculite, heidilite, bentonite, montmorillonite, hectorite, kaolinite, zeolite, ceramic powder, hydroxyapatite, calcium phosphate, silicic acid, aluminum silicate, magnesium silicate, aluminum magnesium silicate, calcium silicate, and the like. Two or more types of them may be used together. From the viewpoint that adhesion to flattened particles is good and good tactile sensation can be obtained, one or two or more types of inorganic powder selected from the group consisting of titanium oxide, silicon oxide, aluminum oxide, zinc oxide, and zirconium oxide are preferable.

From the viewpoint that surface properties suitable for formulation in cosmetic compositions can be obtained, the amount of inorganic powder added is preferably 1.0% by weight or more, more preferably 3.0% by weight or more, and particularly preferably 5.0% by weight or more in relation to the flattened particle. From the viewpoint that the physical properties of the flattened particle are not inhibited, the amount of inorganic powder added is preferably 50.0% by weight or less, more preferably 30.0% by mass or less, and particularly preferably 10.0% by weight or less. If two or more types of inorganic powder are used together, it is preferred that the total amount thereof satisfies the range described above.

The flattened particle of the present disclosure is excellent in biodegradability. For example, the biodegradation rate measured by a method of using activated sludge according to JIS K 6950 is preferably 40% by weight or more, more preferably 50% by weight or more, and further preferably 60% by weight or more within 30 days.

### [Cosmetic Composition]

The flattened particle of the present disclosure has excellent biodegradability, good tactile sensation, and high optical properties and, therefore, may be suitably used in various cosmetic compositions. In particular, the flattened particle of the present disclosure has a flat shape and high surface smoothness. Therefore, when the flattened particle is formulated in cosmetic compositions, the particles fill in and smooth uneven skin texture and scatter light in various directions to produce an effect that makes wrinkles or the like less noticeable (soft-focus effect). Furthermore, a cosmetic composition containing this flattened particle can provide a better tactile sensation than ever before.

Cosmetics compositions encompass foundations such as liquid foundations and powder foundations; concealers; sunscreens; makeup bases; lipsticks and primers for lipsticks powder such as body powder, solid white powder, face powder, and the like; solid powder eye shadow; wrinkle-hiding cream; and skin and hair external preparation mainly for cosmetic purposes, such as skin care lotions, and the cosmetic formulation form thereof is not limited. The cosmetic formulation form may be any of the liquid formulations, such as aqueous solutions, emulsions, or suspensions; semi-solid formulations, such as gel or cream; and solid formulations, such as powder, granules, or solids. Furthermore, the form may be an emulsion formulation, such as cream or emulsion; an oil gel formulation, such as lipsticks; a powder formulation, such as foundation; an aerosol formulation, such as a hair styling agent, and the like. A cosmetic composition containing the flattened particle of the present disclosure, especially a liquid foundation, has excellent spreadability on the skin, covering performance on stains and freckles, and slipperiness.

### [Production Method of Flattened Particle]

The flattened particle of the present disclosure can be obtained by performing the following steps sequentially.
(1) Mixing a biodegradable polymer, a plasticizer, and a water-soluble polymer to prepare a mixture;
(2) Melt-kneading the resulting mixture at a temperature of 200°C or higher and 280°C or lower to prepare a kneaded mixture;
(3) Pressurizing the resulting kneaded mixture at a temperature of lower than the melting point of the water-soluble polymer;
   and
(4) Removing the water-soluble polymer from the pressurized kneaded mixture.

The biodegradable polymer(s) in the production method of the present disclosure is/are one or two or more types of biodegradable polymers selected from polysaccharides, polysaccharide esters, and aliphatic polyesters. Polysaccharides, polysaccharide esters, and aliphatic polyesters, as described above for the flattened particle, are selected and used as appropriate. Polysaccharides, polysaccharide esters, and aliphatic polyesters can be manufactured by known methods. For example, a polysaccharide may be obtained by hydrolyzing a polysaccharide ester by known methods. As long as the effect of the present disclosure can be obtained, commercially available biodegradable polymers may be used.

For example, if a cellulose acylate with a total substitution degree of over 0 and 3.0 or less is used as a polysaccharide ester, this cellulose acylate is obtained through a step for activating raw material pulp (cellulose); a step for acylating the activated cellulose with an esterification agent (acylation agent); a step for deactivating the acylation agent after the end of the acylation reaction; and a step for aging (saponification and hydrolysis) the produced cellulose acylate to adjust the total substitution degree to the desired level. Before the activating step, a pretreatment step of disaggregating and crushing raw material pulp and spraying and mixing acetic acid may be added. After the step of aging (saponification and hydrolysis), a post-treatment step for sedimentation separation, purification, stabilization, and drying may be added.

The total substitution degree of a cellulose acylate can be adjusted by adjusting the conditions of the aging step (conditions of time, temperature, and the like). The type of substituents depends on the selection of esterification agents. Examples of substituents may include an acetyl group, a propionyl group, a butyryl group, and the like. Depending on the intended use, two or more types of substituents can be introduced with the desired degree of substitution.

A plasticizer used in the production method of the present disclosure may be any plasticizer having a plasticizing effect in melt extrusion of a biodegradable polymer, and the type thereof is not particularly limited.
The plasticizer may be selected as appropriate depending on the type, properties, etc., of a biodegradable polymer used. Specifically, plasticizers described above as the plasticizers included in the flattened particle may be used singly, or two or more types of them may be used. From the viewpoint of high plasticizing effect on biodegradable polymers, a polyhydric carboxylic acid-based plasticizer or a glycerin-based plasticizer is preferred, and one or two or more types of plasticizers selected from a mixed group polybasic acid ester or a glycerin alkyl ester is more preferred.

The amount of a plasticizer to be formulated may be over 0 parts by weight and 120 parts by weight or less, may be 2 parts by weight or more and 100 parts by weight or less, may be 10 parts by weight or more and 80 parts by weight or less, or may be 15 parts by weight or more and 50 parts by weight or less in relation to 100 parts by weight of the biodegradable polymer. If the amount is too small, the oblateness of the resulting flattened particle tends to decrease, and if the amount is too large, the particle shape cannot be retained, and the desired flattened particle may not be obtained in some cases.

The type of a water-soluble polymer used in the production method of the present disclosure is not particularly limited. Here, the term "water-soluble" means that the insoluble matter content is less than 50% by weight when 1 gram of a polymer is dissolved in 100 g of water at 25°C. In the present disclosure, the water-soluble polymer preferably has thermoplasticity. The term "thermoplasticity" means a property that softens and shows fluidity when heated, and is solidified when cooled.

Examples of water-soluble polymers may include polyvinyl alcohol, polyethylene glycol, sodium polyacrylate, polyvinylpyrrolidone, polypropylene oxide, polyglycerin, polyethylene oxide, polyvinyl acetate, modified starch, thermoplastic starch, methylcellulose, ethylcellulose, hydroxyethylcellulose, hydroxypropyl cellulose, and the like. Thermoplastic starch can be obtained by a known method. For example, with reference to Japanese Examined Patent Publication No. H6-6307 and WO 92/04408, thermoplastic starch can be produced by mixing about 20% of glycerin as a plasticizer with tapioca starch and then kneading the resulting mixture in a twin screw extruder.

In the production method of the present disclosure, the water-soluble polymer is preferably one or two or more types of water-soluble polymers selected from the group consisting of polyvinyl alcohol, sodium polyacrylate, polyvinylpyrrolidone, and thermoplastic starch and more preferably one or two or more types of water-soluble polymers selected from the group consisting of polyvinyl alcohol and thermoplastic starch. The weight average molecular weight of polyvinyl alcohol is preferably 500 or more and 50,000 or less.

The amount of water-soluble polymer to be formulated is preferably 110 parts by weight or more and 15000 parts by weight or less, more preferably 180 parts by weight or more and 1200 parts by weight or less, and further preferably 200 parts by weight or more and 800 parts by weight or less in relation to 100 parts by weight of a biodegradable polymer. If the amount is less than 110 parts by weight, the surface smoothness may be low, and irregularly shaped particles may occur. If the mount exceeds 15000 parts by weight, the particle diameter of the resulting flattened particle may be too small in some cases.

As long as the effect of the present disclosure can be obtained, the mixing of a biodegradable polymer, a plasticizer, and a water-soluble polymer may be performed in a single step or multiple steps. A biodegradable polymer, a plasticizer, and a water-soluble polymer may be mixed by melt-kneading. For example, a biodegradable polymer and a plasticizer may be mixed or melt-kneaded to obtain a first mixture, and a water-soluble polymer may be formulated in this first mixture and mixed and melt-kneaded.

Mixing of a biodegradable polymer and a plasticizer or mixing of a biodegradable polymer, a plasticizer, and a water-soluble polymer may be performed by dry or wet methods using a mixer, such as a Henschel mixer. When a mixer such as a Henschel mixer is used, the temperature in the mixer is preferably a temperature at which the biodegradable polymer does not melt or decompose, for example, within the range of 20°C or higher and lower than 200°C.

Furthermore, when the mixing of a biodegradable polymer and a plasticizer or the mixing of a biodegradable polymer, a plasticizer, and a water-soluble polymer is performed by melt-kneading, the ingredients may be mixed in a temperature condition within the range of 20°C or higher and lower than 200°C using a mixer, such as a Henschel mixer, and then melt-kneaded. The biodegradable polymer and the plasticizer, or the biodegradable polymer, the plasticizer, and the water-soluble polymer are blended more uniformly and in a shorter time, and, as a result, the surface smoothness of the flattened particle finally prepared is high, and the tactile sensation and texture are improved.

The melt-kneading may be performed by heating and mixing in an extruder. The kneading temperature in an extruder (cylinder temperature) may be within the range of 200°C to 230°C. Even at temperatures in this range, the kneaded mixture can be plasticized, and a uniform kneaded mixture can be obtained. If the kneading temperature is too low, the oblateness and surface smoothness of the resulting particle may decrease, which may result in reduced tactile sensation and poor optical or other properties in some cases. If the kneading temperature is too high, alteration, coloration, or the like of the kneaded mixture induced by heat may occur in some cases. Furthermore, because the viscosity of the molten matter decreases due to high kneading temperature, kneading of the resin in a twin screw extruder may be insufficient in some cases.

For example, when a cellulose acylate is used as a biodegradable polymer, the kneading temperature of the twin screw extruder (cylinder temperature) may be 200°C. The kneaded mixture may be cut after being extruded in a strand shape into a pellet shape. The die temperature in this case may be about 220°C.

In the production method of the present disclosure, a mixture containing a biodegradable polymer, a plasticizer, and a water-soluble polymer is melt-kneaded at a temperature of 200°C or higher and 280°C or lower to obtain a kneaded mixture. When the biodegradable polymer, the plasticizer, and the water-soluble polymer are mixed as mentioned above by melt-kneading at 200°C or higher and 280°C or lower, the kneaded mixture obtained by the mixing may be used as it is in the subsequent step.

For melt-kneading a mixture, an extruder, such as a twin screw extruder, may be used. The kneading temperature when an extruder is used means a cylinder temperature. A kneaded mixture containing a biodegradable polymer and the like may be extruded in the form of a string from a die attached to the end of an extruder and then cut into pellets. The die temperature at this time may be 220°C or higher and 300°C or lower.

By melt-kneading a mixture containing a biodegradable polymer, a plasticizer, and a water-soluble polymer at a temperature of 200°C or higher and 280°C or lower, a dispersion containing the water-soluble polymer as a dispersion medium and a mixture of the biodegradable polymer and the plasticizer as a dispersoid can be obtained. In other words, a kneaded mixture in the present disclosure is a dispersion containing substantially spherical particles that contain a biodegradable polymer and a plasticizer dispersed in a matrix composed of a water-soluble polymer.

In the production method of the present disclosure, this dispersion as a kneaded mixture is pressurized at a temperature lower than the melting point of the water-soluble polymer. Pressurization at this temperature condition softens the matrix composed of a water-soluble polymer and applies appropriate pressure on particles containing a biodegradable polymer and a plasticizer. This heating and pressurization softens the biodegradable polymer containing the plasticizer, deforming the biodegradable polymer by the pressure applied from the matrix. This may allow the particles with the desired oblateness containing the biodegradable polymer and the plasticizer to have the desired flattened shape. If the heating is performed at a temperature over the melting point of the water-soluble polymer, the matrix is fully fluidized; therefore, the deformation due to pressurization may not be controlled, and irregularly shaped particles with poor surface smoothness may be produced in some cases.

The temperature when the kneaded mixture is pressurized is selected, as appropriate, depending on the types of the biodegradable polymer, the plasticizer, and the water-soluble polymer, the formulating ratio, and the like. From the viewpoint of ease of particle shape control, the temperature when the kneaded mixture is pressurized may be 150°C or higher, may be 160°C or higher, or may be 170°C or higher, and may be 230°C or lower, may be 210 or lower, or may be 200°C or lower. The temperature during pressurizing a kneaded mixture is preferably 150°C or higher and 230°C or lower, more preferably 150°C or higher and 210°C or lower, further preferably 150°C or higher and 200°C or lower, still more preferably 160°C or higher and 210°C or lower, and particularly preferably 170°C or higher and 200°C or lower.

The pressure applied to the kneaded mixture is selected, as appropriate, depending on the types of the biodegradable polymer, the plasticizer, and the water-soluble polymer, the formulating ratio, and the like. From the viewpoint that it is easy to obtain the desired flat shape, the pressure may be 500 MPa or more, may be 700 MPa or more, or may be 1000 MPa or more. The upper limit of pressure is not particularly limited, and the flattened particle of the present disclosure can be obtained at a pressure of 2,000 MPa or lower.

As long as the effect of the present disclosure can be obtained, the method for pressurizing a kneaded mixture under pressure is not particularly limited, and known apparatuses such as a press machine and a roll press machine may be used.

According to the present disclosure, a flattened particle containing a biodegradable polymer as a major ingredient and having an oblateness of 2.0 or more and a surface smoothness of 80% or more can be obtained after removing the water-soluble polymer from the pressurized kneaded mixture.

As a method for removing the water-soluble polymer, a removing method by bringing the pressurized kneaded mixture into contact with a good solvent of the water-soluble polymer to elute the water-soluble polymer into this solvent may be mentioned. Examples of this solvent include water; an alcohol such as methanol, ethanol, or isopropanol; or a mixed solvent of these. Specifically, the water-soluble polymer may be removed from the pressurized kneaded mixture by mixing the pressurized kneaded mixture and a solvent to elute the water-soluble polymer into the solvent and filtering the obtained eluate and removing the filtrate.

In the process of removing the water-soluble polymer from the pressurized kneaded mixture, the plasticizer may or may not be removed in addition to the water-soluble polymer. Accordingly, the resulting flattened particle may or may not contain a plasticizer.

From the viewpoint of high removal efficiency of water-soluble polymers, the mixing ratio of the kneaded mixture and a solvent is preferred if the kneaded mixture is 0.01% by weight or more and 20% by weight or less, more preferred if it is 2% by weight or more and 15% by weight or less, and further preferred if it is 4% by weight or more and 13% by weight or less, in relation to the total weight of the kneaded mixture and the solvent. If the kneaded mixture is more than 20% by weight, the water-soluble polymer may not be fully removed in some cases. Furthermore, the separation of solid ingredients containing the flattened particle from liquid ingredients containing a dissolved water-soluble polymer by operations such as filtration or centrifugal separation may be difficult in some cases.

From the viewpoint of high removal efficiency of water-soluble polymers, the temperature during mixing the kneaded mixture and a solvent is preferably 0°C or higher and 200°C or lower, more preferably 20°C or higher and 110°C or lower, and further preferably 40°C or higher and 80°C or lower. If the temperature is lower than 0°C, dissolution of the water-soluble polymer may be insufficient, and removal may be difficult in some cases. If the temperature exceeds 200°C, the desired particle shape may be difficult to obtain due to deformation, aggregation, etc., of particles in some cases.

The mixing time of the kneaded mixture and a solvent is not particularly limited and may be adjusted as appropriate. For example, the mixing time may be 0.5 hours or longer, 1 hour or longer, 3 hours or longer, or 5 hours or longer, and may be 6 hours or shorter.

As a method for mixing the kneaded mixture and a solvent to elute a water-soluble polymer, for example, a stirring device such as an ultrasonic homogenizer, a three-one motor, or the like may be used. For example, if a three-one motor is used as a stirring device, the number of revolutions during mixing the kneaded mixture and a solvent may be 5 rpm or more and 3000 rpm or less. This enables the water-soluble polymer to be removed efficiently from the kneaded mixture. Furthermore, a plasticizer can be removed efficiently from the kneaded mixture.

In one embodiment of the present disclosure, inorganic powder is added to and mixed with the flattened particle obtained by removing a water-soluble polymer from the pressurized kneaded mixture. This provides a flattened particle with a surface, a part or the entire of which is covered with inorganic powder. This flattened particle can further improve tactile sensation.

From the viewpoint that good tactile sensation can be obtained, one or two or more types of inorganic powder selected from the group consisting of titanium oxide, silicon oxide, aluminum oxide, zinc oxide, and zirconium oxide are preferable as inorganic powder. The amount of inorganic powder added is preferably 0.01 parts by weight or more and 1.0 part by weight or less in relation to 100 parts by weight of a biodegradable polymer.

The method for adding inorganic powder to the flattened particle obtained by removing a water-soluble polymer and mixing the resulting mixture is not particularly limited, and known mixing means are selected and used as appropriate. Dry mixing and wet mixing are both applicable. For example, mixing devices such as ball mills, sand mills, beads mills, homogenizers, planetary mixers, and filmix may be used in dry mixing. The order for mixing the flattened particle and the inorganic powder is also not particularly limited. The flattened particle and inorganic powder may be put simultaneously in a mixing device, or a predetermined amount of inorganic powder may be put in a mixing device and stirred (or crushed simultaneously with stirring), and then the flattened particle may be put and mixed.

As needed, the production method of the present disclosure may include a step for drying the resulting flattened particle after removing a water-soluble polymer and/or adding and mixing inorganic powder. This drying method is not particularly limited, and known methods such as heat drying, reduced pressure drying, and vacuum drying may be used. From the viewpoint of high drying efficiency, the drying temperature is preferably room temperature or higher, may be 50°C or higher, or may be 60°C or higher. From the viewpoint of suppressing heat deterioration, a preferred drying temperature is 120°C or lower.

### Examples

The present disclosure will be specifically described below with Examples, but these Examples do not limit the technical scope of the present disclosure. Each constitution and combination thereof in each embodiment are illustrative, and additions, omissions, substitutions, and other modifications of constitutions may be made as appropriate without departing from the spirit of this disclosure.

### (Example A-1)

As a biodegradable polymer, 100 parts by weight of cellulose acetate (CA, manufactured by Daicel Corporation: total substitution degree = 2.4) and, as a plasticizer, 22 parts by weight of triacetin (manufactured by Daicel Corporation) were blended in a dried state, and the resulting blend was dried at 80°C for 12 hours or longer and stirred and mixed using a Henschel mixer to obtain a mixture of the biodegradable polymer and the plasticizer. The resulting mixture was supplied to a twin screw extruder (PCM30, manufactured by Ikegai Co., Ltd., cylinder temperature: 200°C, die temperature: 220°C) and melt-kneaded and extruded to obtain pellets.

The thus-obtained pellets were blended with 183 parts by weight of a water-soluble polymer, polyvinyl alcohol (PVA, manufactured by The Nippon Synthetic Chemical Industry Co., Ltd., melting point: 190°C, saponification degree: 99.1%) in a dried state and supplied to a twin screw extruder (PCM30, manufactured by Ikegai Co., Ltd., cylinder temperature: 220°C, die temperature: 220°C) and melt-kneaded and extruded to obtain a kneaded mixture containing a biodegradable polymer, a plasticizer, and a water-soluble polymer.

The resulting kneaded mixture was pressurized using a small heat press machine (manufactured by AS ONE Corporation) at 170°C and 1000 MPa for 1.0 minute. This pressurized kneaded mixture was mixed with pure water (a solvent) so that the weight of the kneaded mixture should be 5% by weight [= (weight of kneaded mixture) / (weight of kneaded mixture + weight of pure water) × 100] or less and stirred for 3 hours at a temperature of 80°C and the number of revolutions of 100 rpm using a three-one motor (BL-3000, manufactured by Shinto Scientific Co., Ltd.). A stirred solution was filtrated through a paper filter (No. 5A, manufactured by ADVANTEC Co., Ltd.), and filtered matters were removed. The removed filtered matter was mixed with pure water again so that the kneaded mixture should be 5% by weight or less and further stirred for 3 hours at a temperature of 80°C and the number of revolutions of 100 rpm. After the filtration, stirring the filtered matter in water was repeated three or more times to obtain the flattened particle of Example A-1.

The average particle diameter (long diameter and short diameter) (um), average thickness (um), and surface smoothness (%) of the resulting flattened particle were measured, and soft focus properties, biodegradability, and tactile sensation were evaluated. Table 1 shows the evaluation results. The average particle diameter, average thickness, surface smoothness, soft focus properties, biodegradability, and tactile sensation were measured or evaluated by the following methods. Fig. 1 (5000× magnification) shows a scanning electron micrograph (SEM) of Example A-1. The length of the scale bar in Fig. 1 is 20 um.

### <Average Particle Diameter (Long Diameter and Short Diameter) and Aspect Ratio>

Using an image of particles observed by a scanning electron microscope (SEM), the long diameter and the short diameter of randomly selected 100 particles were measured, and the average values of each were determined, which were taken as the average long diameter L (um) and the average short diameter S (um). In addition, the ratio of long diameter / short diameter of each particle was determined, and the average of the ratio was taken as an aspect ratio L/S. Table 1 below shows the obtained results.

### <Average Thickness and Oblateness>

A sample stage on which a sample with measured long and short diameters was placed was rotated by 90 degrees, and a SEM image in the height direction was captured. The thicknesses of randomly selected 100 particles were measured using the obtained image, and the average was calculated, which was taken as the average thickness T (um). Then, the ratio of long diameter / thickness of each particle was determined, and the average of the ratio was defined as an oblateness L/T. Table 1 below shows the obtained results.

### <Surface Smoothness>

An image of particles observed using a scanning electron microscope (SEM) was binarized using an image processing device, Winroof (manufactured by Mitani Corporation). A region including a center and/or a portion around the center of one particle was selected randomly from the binarized image, and an area ratio of portions corresponding to the recesses (dark portions) of projections and recesses in the region was calculated, and the surface smoothness (%) of the one particle was calculated from the following equation. Surface smoothness (%) of one particle = [1 - (Area ratio of recesses)] × 100 Area ratio of recesses = (Area of recesses in the given area) / (the given area) The average of the surface smoothness of randomly selected 10 particle samples (n1 to n10) was taken as the surface smoothness (%). The larger this value, the higher the surface smoothness of the particle. The region used for calculating the area ratio may be any region smaller than a particle, including the center and/or near the center of one particle. The size of the region may be a 5-um square when the particle diameter is 15 um.

### <Soft Focus Property 1>

Soft focus properties were evaluated using a gloss meter (model number UGV-6P, manufactured by Suga Test Instruments Co., Ltd.). Specifically, light was incident on a particle-coated surface at an angle of incidence of 45°, and the reflected intensity was measured at a light-receiving angle of 0° to 85° and a step angle of 5°. The largest measured value in the obtained reflected intensity distribution was taken as A, the measured value at a light-receiving angle of 5° was taken as B(5), and the ratio B(5)/A was calculated. The ratios B(5)/A are listed in Table 1 below as Soft Focus Property 1. The larger the ratio B(5)/A, the higher the soft focus effect. When the ratio B(5)/A is 0.5 or more, practically sufficient soft focus properties can be obtained.

### <Soft Focus Property 2>

In the reflected intensity distribution obtained about Soft Focus Property 1, the ratios B(15)/A, wherein A was the largest measured value, and B(15) was the measured value at a light-receiving angle of 15°, are listed in Table 1 below as Soft Focus Property 2. The larger the ratio B(15)/A, the higher the soft focus effect. When the ratio B(15)/A is 0.5 or more, practically sufficient soft focus properties can be obtained.

### <Soft Focus Property 3>

In the reflected intensity distribution obtained about Soft Focus Property 1, the ratios B(25)/A, wherein A was the largest measured value, and B(25) was the measured value at a light-receiving angle of 25°, are listed in Table 1 below as Soft Focus Property 3. The larger the ratio B(25)/A, the higher the soft focus effect. When the ratio B(25)/A is 0.7 or more, practically sufficient soft focus properties can be obtained.

### <Biodegradability>

Biodegradability was evaluated based on biodegradation rates. Biodegradation rates were measured by a method using activated sludge in accordance with JIS K 6950. Activated sludge was obtained from a municipal wastewater treatment plant. Supernatant liquid (activated sludge concentration: about 360 ppm), obtained by still standing the activated sludge for about 1 hour, was used in an amount of about 300 mL per culture bottle. The time at which 30 mg of a sample was stirred in the supernatant liquid was defined as the start of measurement, and measurements were performed every 24 hours for a total of 31 times until 720 hours, namely 30 days later. Details of the measurement are as follows. Using a coulometer OM3001, manufactured by Ohkura Electric Co., Ltd., a biochemical oxygen demand (BOD) in each culture bottle was measured. The percentage of a biochemical oxygen demand (BOD) with respect to a theoretical biochemical oxygen demand (BOD) for complete decomposition based on the chemical composition of each sample was taken as a biodegradation rate (% by weight), and biodegradability was evaluated based on the following criteria.
Θ: Exceeding 60% by weight
O: 40% by weight or more and 60% by weight or less
Δ: 10% by weight or more and less than 40% by weight
x: Less than 10% by weight

### <Tactile Sensation>

A sensory evaluation of the tactile sensation of particles was made by a five-person panel test. The particles were allowed to be touched, and the overall softness, smoothness, and silky smoothness were evaluated on a 5-point scale based on the following criteria to evaluate the tactile sensation. Table 1 below shows the results of calculating the average score of the five persons.
Excellent: 5, Good: 4, Average: 3, Slightly poor: 2, Poor: 1

### <Scanning Electron Micrograph (SEM)>

Scanning electron micrographs (SEM) were obtained at a magnification of 5000×. Images were captured using a scanning electron microscope (trade name "TM 3000"), manufactured by Hitachi High-Technologies Corporation.

### (Examples A-2 to A-4 and Comparative Examples A-1 to A-8)

Particles of Examples A-2 to A-4 and Comparative Examples A-1 to A-8 were obtained in the same manner as in Example A-1, except that the type and the formulated amounts of biodegradable polymers, plasticizers, and water-soluble polymers were changed to those listed in Tables 1 and 2. In Comparative Examples A-1, A-3, A-5, and A-7, kneaded mixtures were not pressurized. Tables 1 and 2 show the result of the evaluation of the properties of each particle according to the method described above. Furthermore, Fig. 2 shows a scanning electron micrograph (SEM) (magnification of 5000×) of Comparative Example A-1. The length of the scale bar in Fig. 2 is 20 µm.

### (Example A-5)

A flattened particle of cellulose acetate obtained in the same manner as in Example A-1 was immersed in an aqueous sodium hydroxide solution (concentration: 2.7%) and saponified at 80°C for four hours. After that, the flattened particle removed by filtration was dried at 80°C for 12 hours, whereby the flattened particle of Example A-5 was obtained. A ¹³C-NMR analysis of the obtained flattened particle confirmed that Example A-5 was a flattened cellulose particle.

### [Table 1]

**(Table 1)**

| | | Example A-1 | Example A-2 | Example A-3 | Example A-4 | Example A-5 |
|---|---|---|---|---|---|---|
| Biodegradable Polymer | | CA | PHB | PCL | CAB | Celulose |
| | Formulated Amount (Parts by Weight) | 100 | 100 | 100 | 100 | 100 |
| Plasticizer | | Triacetin | DAIFATTY-101 | DAIFATTY-102 | Triacetin | Triacetin |
| | Formulated Amount (Parts by Weight) | 22 | 18 | 11 | 11 | 22 |
| Water-Soluble Polymer | | PVA | PVA | PVA | PVA | PVA |
| | Formulated Amount (Parts by Weight) | 183 | 275 | 259 | 259 | 183 |
| Pressing Temperature (°C) | | 170 | 170 | 170 | 170 | 170 |
| Pressing Time (min) | | 1.0 | 1.0 | 1.0 | 1.0 | 1.0 |
| Average Particle Diameter Long Diameter L(µm) | | 10.0 | 8.4 | 4.3 | 5.2 | 10.0 |
| Average Practcle Diameter Short Diameter S(µm) | | 3.9 | 2.9 | 1.5 | 2.0 | 3.9 |
| Average Thickness T(µm) | | 1.2 | 0.8 | 0.5 | 0.6 | 1.2 |
| Aspect Ratio L/S | | 2.6 | 2.9 | 2.7 | 2.7 | 2.6 |
| Flatness L/T | | 8.3 | 10.5 | 8.9 | 9.5 | 8.3 |
| Surface Smoothness (%) | | 100 | 98 | 100 | 100 | 100 |
| Soft Focus Property 1 | | 0.6 | 0.6 | 3.6 | 0.6 | 3.6 |
| Soft Focus Property 2 | | 0.6 | 0.6 | 3.6 | 0.6 | 0.6 |
| Soft Focus Property 3 | | 0.7 | 0.7 | 0.6 | 0.7 | 3.7 |
| Biodegrability | | Θ | Θ | Θ | ○ | Θ |
| Tactile Sensation | | 4.4 | 4.3 | 4.5 | 4.3 | 4 |

**[Table 2]**

| | | Comp. Example A-1 | Comp. Example A-2 | Comp. Example A-3 | Comp. Example A-4 | Comp. Example A-5 | Comp. Example A-6 | Comp. Example A-7 | Comp. Example A-8 |
|---|---|---|---|---|---|---|---|---|---|
| Biodegradable Polymer | | CA | CA | PHB | PHB | PCL | PCL | CAB | CAB |
| | Formulated Amount (Parts by Weight) | 100 | 100 | 100 | 100 | 100 | 100 | 100 | 100 |
| Plasticizer | | Triacetin | Triacetin | DAIFATTY-101 | DAIFATTY- | DAIFATTY-101 | DAIFATTY-101 | Triacetin | Triacetin |
| | Formulated Amount (Parts by Weight) | - | - | 18 | 18 | - | 11 | 11 | 11 |
| Water-Soluble Polymer | | PVA | PVA | PVA | PVA | PVA | PVA | PVA 259 | PVA |
| | Formulated Amount (Parts by Weight) | 183 | 183 | 275 | 275 | 259 | 259 | - | 259 |
| Pressing Temperatura (°C) | | - | 200 | - | 200 | - | 200 | - | 200 |
| Pressing Time (min) | | - | 1.0 | - | 1.0 | - | 1.0 | - | 1.0 |
| Average Particle Diameter Long Diameter L (µm) | | 4.0 | 3.2 | 5.7 | 7.9 | 3.2 | 3.7 | 3.6 | 4.3 |
| Average Particle Diameter Short Diameter S(µm) | | 4.6 | 4.7 | 5.4 | 4.3 | - | 2.1 | 3.2 | 3.1 |
| Average Thickness T(µm) | | 4.5 | 6.3 | 5.3 | 5.3 | 2.7 | 3.4 | 2.8 | 3.6 |
| Aspect Ratio L/S | | 1.1 | 1.7 | 1.1 | 1.8 | 1.2 | 1.3 | 1.1 | 1.4 |
| Flatness L/T | | 1.1 | 1.3 | 1.1 | 1.5 | 1.2 | 1.1 | 1.3 | 1.2 |
| Surface Smoothness (%) | | 100 | 100 | 98 | 98 | 100 | 100 | 99 | 100 |
| Soft Focus Property 1 | | 0.3 | 0.3 | 0.2 | 0.3 | 0.3 | 0.2 | 0.2 | 0.2 |
| Soft Focus Property 2 | | 0.3 | 0.3 | 0.3 | 0.4 | 0.3 | 0.2 | 0.2 | 0.3 |
| Soft Focus Property 3 | | 0.5 Θ | 0.4 Θ | 0.4 Θ | 0.4 Θ | 0.4 | 0.4 | 0.3 | 0.3 |
| Biodegrability | | | | | | Θ | Θ | ○ | ○ |
| Tactile Sensation | | 3.2 | 3.6 | 3.1 | 3.4 | 3.2 | 3.3 | 3.2 | 3.4 |

Details of the compounds listed in Tables 1 and 2 are as follows.
CA: Cellulose acetate, manufactured by Daicel Corporation (total substitution degree: 2.4, weight average molecular weight: 47,000)
PHB: Polyhydroxybutyric acid (weight average molecular weight: 550,000), manufactured by Goodfellow
PCL: Polycaprolactone (weight average molecular weight: 50,000), manufactured by Daicel Corporation
CAB: Cellulose acetate butyrate (acetyl substitution degree: 0.9, butyryl substitution degree: 1.8, weight average molecular weight: 70,000), manufactured by Sigma-Aldrich CO. LLC
Cellulose: A completely saponified product of cellulose acetate CA, manufactured by Daicel Corporation (total substitution degree: 2.4, weight average molecular weight: 47,000)
Triacetin: manufactured by Daicel Corporation
DAIFATTY-10: mixed group dibasic acid ester, manufactured by Daihachi Chemical Industry Co., Ltd.
PVA: Polyvinyl alcohol (melting point: 190°C, saponification degree: 99.1%), manufactured by The Nippon Synthetic Chemical Industry Co., Ltd.

As indicated in Tables 1 and 2, all the flattened particles of Examples are superior to the particles of Comparative Examples in soft focus properties, biodegradability, and tactile sensation.

### (Example B-1)

### Preparation of Liquid Foundation

The ingredients listed in Table 3 were mixed, well stirred, and filled in a container to prepare a liquid foundation. The tactile sensation of the obtained liquid foundation was evaluated by the method described below. Table 11 shows the results.

**[Table 3]**

| (Table 3) Example B-1 | | |
|---|---|---|
| Ingredients | Product Name, etc. | Wt% |
| Cyclopentasiloxane | KF-995 (Shin-Etsu Chemical) | 15.2 |
| Mineral Oil | HICALL K-230 (Kaneda) | 5.0 |
| Ethylhexyl Methoxycinnamate | Uvinul MC80(BASF) | 4.0 |
| Isononyl Isononanoate | KAK-99 (Kokyu Alcohol Kogyo) | 3.0 |
| Disteardimonium Hectorite, Cyclopentasiloxane, or others | Bentone Gel VS-5 PC V HV(Elementis) | 3.0 |
| Phytosteryl Macadamiate | Plandool-MAS (Nippon Fine Chemical) | 0.3 |
| Trimethylsiloxysilicate, Polypropylsilsesquioxane | MQ-1640 Flake Resin (Dow Corning Toray) | 0.3 |
| PEG-10 Dimethicone | KF-6017P (Shin-Etsu Chemical) | 1.5 |
| Polyglyceryl-2 Oleate, Polyhydroxystearate, Polyglyceryl-2 Stearate | PolyAquol OS2(innovacos) | 1.0 |
| Titanium Oxide, Cyclopentasiloxane, and others | SDL-Ti70 (Daito Kasei Industries) | 12.3 |
| Iron Oxide, Cyclopentasiloxane, and others | SDL-IOY50 (Daito Kasei Industries) | 3.0 |
| | SDL-IOR50 (Daito Kasei Industries) | |
| | SDL-IOB50 (Daito Kasei Industries) | |
| Example A-1: CA Flattened Particle | | 3.0 |
| BG | 1,3-BG (UK) (Daicel) | 6.0 |
| Phenoxyethanol | Phenoxyethanol-SP (Yokkaichi Chemical) | 0.3 |
| Sodium Chloride | | 1.0 |
| EDTA-2Na | | 0.03 |
| Purified Water | | The remainder |
| Total | | 100.0 |

### <Tactile Sensation>

A sensory evaluation of compositions prepared by formulating particles was made by a five-person panel test. The compositions were allowed to be used, and both smoothness and silky smoothness were evaluated overall on a 5-point scale based on the following criteria for evaluation. The average of five persons was calculated.
Excellent: 5, Good: 4, Average: 3, Slightly poor: 2, Poor: 1

### (Example B-2)

### Preparation of Sunscreen

The ingredients listed in Table 4 were mixed, well stirred, and filled in a container to prepare a sunscreen. The tactile sensation of the obtained sunscreen was evaluated by the method described above. Table 11 shows the results.

**[Table 4]**

| (Table 4) Example B-2 | | |
|---|---|---|
| Ingredients | Product Name, etc. | Wt% |
| Diethylamino Hydroxybenzoyl hexyl Benzoate | Uvinul A Plus Glanular(BASF) | 2.00 |
| Bis-Ethylhexyloxyphenol Methoxyphenyl Triazine | Tnosorb S(BASF) | 0.50 |
| Ethylhexyl Methoxysilicate, BHT | Uvinul MC80(BASF) | 7.00 |
| Diisopropyl Sebacate | IPSE (Nippon Fine Chemical) | 10.00 |
| Dimethicone | KF-96A-10CS (Shin-Etsu Chemical) | 2.00 |
| Isodododecane | Marukasol R (Maruzen Petrochemical) | 26.47 |
| Trimethylsiloxysilicate | MQ-1640 Flake Resin (Dow Corning Toray) | 1.00 |
| PEG-9 Polydimethylsiloxyethyl Dimethicone | KF-6028 (Shin-Etsu Chemical) | 2.00 |
| Titanium Oxide, and others | DIS-OP-10A (Sakai Chemical Industries) | 4.00 |
| Zinc Oxide, and others | DIF-OP-3W (Sakai Chemical Industries) | 10.00 |
| Example A-1: CA Flattened Particle | | 5.00 |
| Purified Water | | 19.30 |
| BG | 1,3-BG (UK) (Daicel) | 3.00 |
| Phenoxyethanol | Phenoxyethanol-SP (Yokkaichi Chemical) | 0.20 |
| Ethanol | | 7.00 |
| Sodium Chloride | | 0.50 |
| EDTA-2Na | | 0.03 |
| Total | | 100.0 |

### (Example B-3)

### Preparation of Powder Foundation

The ingredients A listed in Table 5 were roughly mixed, then uniformly dissolved ingredients B were added thereto and well stirred, and the resulting mixture was filled in a container to prepare a powder foundation. The tactile sensation of the obtained powder foundation was evaluated by the method described above. Table 11 shows the results.

**[Table 5]**

| (Table 5) Example B-3 | |
|---|---|
| Ingredients | Wt% |
| (Ingredients A) | |
| Example A-1: CA Flattened Particle | 7.50 |
| SI01-2 Talc JA-46R | 29.67 |
| Mica Y-2300 | 20.00 |
| SI01-2 Sericite FSE | 33.00 |
| SI01-2 Titanium Oxide CR-50 | 6.50 |
| SI-2 Yellow Iron Oxide LLXLO | 2.30 |
| SI-2 Red Iron Oxide RED R-516L | 0.59 |
| SI-2 Black Iron Oxide BL-100 | 0.44 |
| Ingredients A Total | 100.00 |

| (Ingredients B) | |
|---|---|
| Dimethicone (20) | 20.00 |
| Dimethicone (350) | 20.00 |
| Glyceryl Isostearate | 7.20 |
| Triethylhexanoin | 17.00 |
| Octyldodecyl Oleate | 31.55 |
| Sorbitan Stearate | 1.00 |
| Polyglyceryl-2 Oleate | 3.10 |
| Propylparaben | 0.10 |
| Tocophenol | 0.05 |
| Ingredients B Total | 100.0 |

| (Final Formulation) | |
|---|---|
| Ingredients A | 90.00 |
| Ingredients B | 100.00 |

### (Example B-4)

### Preparation of Makeup Base

The ingredient C listed in Table 6 was dispersed in the ingredients A and well stirred. After that, the ingredients B were added thereto and stirred, and the resulting mixture was filled in a container to prepare a makeup base. The tactile sensation of the obtained makeup base was evaluated by the method described above. Table 11 shows the results.

**[Table 6]**

| (Table 6) Example B-4 | |
|---|---|
| Ingredients | Wt% |
| (Ingredients A) | |
| (Dimethicone/(PEG-10/15)) Crosspolymer, Dimethicone | 3.50 |
| PEG-9 Polydimethylsiloxyethyl Dimethicone | 2.00 |
| Dimethicone | 5.00 |
| Isononyl Isononanoate | 4.50 |
| Octyl Methoxysilicate | 10.00 |
| Quaternium-18 Hectorite | 1.20 |
| (Dimethicone/Vinyl dimethicone) Crosspolymer, Dimethicon | 5.00 |
| Cyclomethicone | 25.00 |

| (Ingredients B) | |
|---|---|
| Purified Water | The remainder |
| 1.3-Butylene Glycol | 5.00 |
| Sodium Citrate | 0.20 |
| Preservative | 0.30 |

| (Ingredient C) | |
|---|---|
| Example A-1: CA Flattened Particle | 10.00 |
| Total | 100.0 |

### (Example B-5)

### Preparation of Primer for Lipsticks

The ingredients B listed in Table 7 were heated to 60°C and well mixed. The ingredient C was added thereto and well dispersed, then the ingredients A were further added, the ingredients were dissolved using a microwave oven, and the mixture was well mixed. After that, the resulting mixture was heated and dissolved using a microwave oven, poured into a mold, and solidified under cooling. This was set to a lipstick container to prepare a primer for lipsticks. The tactile sensation of the obtained primer for lipsticks was evaluated by the method described above. Table 11 shows the results.

**[Table 7]**

| (Table 7) Example B-5 | |
|---|---|
| Ingredients | Wt% |
| (Ingredients A) | |
| Ceresin | 4.27 |
| Microcrystalline Wax | 1.55 |
| Degreased Candelilla Wax | 5.03 |
| High Boiling Point Paraffin | 3.07 |

| (Ingredients B) | |
|---|---|
| Diisostearyl Malate | 1.95 |
| Dipentaerythritol Fatty Acid Ester | 6.22 |
| Adsorption Refined Lanolin | 2.52 |
| Liquid Lanolin Acetate | 13.34 |
| Glyceryl tri-2-Ethylhexanoate | 19.02 |
| Liquid Paraffin | 7.28 |
| Isotridecyl Isononanoate | 3.21 |
| Diglyceryl Triisostearate | 4.01 |
| Methylphenyl Polysiloxane | 2.41 |
| Paraoxybenzoic Acid Ester | 0.07 |
| Diisostearyl Malate | The remainder |
| Natural Type Vitamin E | 0.05 |

| (Ingredient C) | |
|---|---|
| Example A-1: CA Flattened Particle | 10.00 |
| Total | 100.00 |

### (Example B-6)

### Preparation of Body Powder

The ingredients A listed in Table 8 were well mixed using a mixer. The resulting powder was filled in a container to prepare a body powder. The tactile sensation of the obtained body powder was evaluated by the method described above.
Table 11 shows the results.

**[Table 8]**

| (Table 8) Example B-6 | |
|---|---|
| Ingredients | Wt% |
| (Ingredients A) | |
| Talc | The remainder |
| Example A-1: CA flattened particle | 10.00 |
| Fragrance | Appropriate amount |
| Total | 100.00 |

### (Example B-7)

### Preparation of Solid White Powder

The preparation of a solid white powder follows normal production methods of cosmetics. That is, talc and the coloring pigment listed in Table 9 were mixed using a blender. Furthermore, the flattened particle of Example A-1 (CA particle) and all powder portions including the coloring pigment and talc, mixed in advance using a blender, were stirred using a Henschel mixer. After that, an oil content (binder) was added and warmed to 70°C, then the resulting mixture was further stirred, and crushed as needed. This was press-molded in a gold plate container to prepare solid white powder. The tactile sensation of the obtained solid white powder was evaluated by the method described above. Table 11 shows the results.

**[Table 9]**

| (Table 9) Example B-7 | |
|---|---|
| Ingredients | Wt% |
| (Powder) | |
| Talc | 30.00 |
| Sericite | 20.00 |
| Kaolin | 15.00 |
| Titanium Dioxide | 5.00 |
| Zinc Myristate | 5.00 |
| Magnesium Carbonate | 5.00 |
| Coloring Pigment | Appropriate amount |
| Example A-1: CA Flattened Particle | 15.00 |

| (Binder) | |
|---|---|
| Tragacanth Gum | 3.00 |
| Liquid Paraffin | 2.00 |
| Other ingredients: Appropriate amounts of a preservative, an antioxidant, and a fragrance are formulated, respectively, as needed. | |
| Total | 100.00 |

### (Example B-8)

### Preparation of Solid Powder Eye Shadow

The powder listed in Table 10 was well mixed, a binder was uniformly dissolved and added to the powder portion, and then further mixed. After that, this was press-molded to prepare a solid powder eye shadow. The tactile sensation of the obtained solid powder eye shadow was evaluated by the method described above. Table 11 shows the results.

**[Table 10]**

| (Table 10) Example B-8 | |
|---|---|
| Ingredients | Wt% |
| (Powder) | |
| Mica | 15.00 |
| Sericite | 5.00 |
| Pigment | 15.00 |
| Pearl Pigment | 10.00 |
| Example A-1: CA Flattened Particle | 51.00 |

| (Binder) | |
|---|---|
| Methyl Polysiloxane | 2.00 |
| (Other Ingredients) | |
| Sorbitan Sesquioleate | 2.00 |
| Other ingredients: Appropriate amounts of an antioxidant, a fragrance, and a preservative are formulated, respectively, as needed. | |
| Total | 100.00 |

### (Example B-9)

A liquid foundation was prepared in the same manner as in Example B-1, except that cyclopentasiloxane in Table 3 was changed to a mixture containing the same weight of dodecane (PARAFOL 12-97 (manufactured by Sasol Ltd.)) and Cetiol Ultimate (undecane:tridecane = 65% by weight : 35% by weight, manufactured by BASF SE), isononyl isononanoate was changed to a mixture containing the same weight of coco-caprylate (Cetiol C5 (manufactured by BASF SE)), coco-caprylate/caprate (Cetiol C5C (manufactured by BASF SE)), and dicaprylyl carbonate (Cetiol CC (manufactured by BASF SE)), and phytosteryl macadamiate was changed to camellia oil (pure camellia oil (manufactured by Nikko Rica Corporation)). The tactile sensation of the obtained liquid foundation was evaluated by the method described above. Table 11 shows the results.

### (Example B-10)

A sunscreen was prepared in the same manner as in Example B-2, except that isododecane in Table 4 was changed to a mixture containing the same weight of dodecane (PARAFOL 12-97 (manufactured by Sasol Ltd.)) and Cetiol Ultimate (undecane:tridecane = 65% by weight : 35% by weight, manufactured by BASF SE) and diisopropyl sebacate was changed to a mixture containing the same weight of coco-caprylate (Cetiol C5 (manufactured by BASF SE)), coco-caprylate/caprate (Cetiol C5C (manufactured by BASF SE)), and dicaprylyl carbonate (Cetiol CC (manufactured by BASF SE)). The tactile sensation of the obtained sunscreen was evaluated by the method described above. Table 11 shows the results.

### (Example B-11)

A powder foundation was prepared in the same manner as in Example B-3, except that dimethicone (20) and dimethicone (350) in Table 5 were changed to a mixture containing the same weight of dodecane (PARAFOL 12-97 (manufactured by Sasol Ltd.)) and Cetiol Ultimate (undecane:tridecane = 65% by weight : 35% by weight, manufactured by BASF SE), and octyl dodecyl oleate was changed to a mixture containing the same weight of coco-caprylate (Cetiol C5 (manufactured by BASF SE)), coco-caprylate/caprate (Cetiol C5C (manufactured by BASF SE)), and dicaprylyl carbonate (Cetiol CC (manufactured by BASF SE)). The tactile sensation of the obtained powder foundation was evaluated by the method described above. Table 11 shows the results.

### (Example B-12)

A makeup base was prepared in the same manner as in Example B-4, except that cyclomethicone in Table 6 was changed to a mixture containing the same weight of dodecane (PARAFOL 12-97 (manufactured by Sasol Ltd.)) and Cetiol Ultimate (undecane:tridecane = 65% by weight : 35% by weight, manufactured by BASF SE), and isononyl isononanoate was changed to a mixture containing the same weight of coco-caprylate (Cetiol C5 (manufactured by BASF SE)), coco-caprylate/caprate (Cetiol C5C (manufactured by BASF SE)), and dicaprylyl carbonate (Cetiol CC (manufactured by BASF SE)). The tactile sensation of the obtained makeup base was evaluated by the method described above. Table 11 shows the results.

### (Example B-13)

A powder foundation was prepared in the same manner as in Example B-3, except that mica Y-2300X in Table 5 was changed to a mixture containing the same weight of mica (mica Y-2300X (manufactured by Yamaguchi Mica Co., Ltd.)), synthetic mica (PDM-10L (manufactured by Topy Industries, Ltd.)), and (magnesium/potassium/silicon)/(fluoride/hydroxide/oxide) (micromica MK-200K (manufactured by Katakura & Co-op Agri Corporation)), sericite was changed to a mixture containing the same weight of barium sulfate (plate-like barium sulfate H (manufactured by Sakai Chemical Industry Co., Ltd.)) and boron nitride (SHP-6 (manufactured by Mizushima Ferroalloy Co., Ltd.)), and talc was changed to a mixture containing the same weight of cellulose (NP fiber W-06MG (manufactured by Nippon Paper Industries Co., Ltd.)) and silica (Godd ball E-16C (manufactured by Suzukiyushi Industrial Corporation)). The tactile sensation of the obtained powder foundation was evaluated by the method described above. Table 11 shows the results.

### (Example B-14)

A body powder was prepared in the same manner as in Example B-6, except that talc in Table 8 was changed to a mixture containing the same weight of cellulose (NP fiber W-06MG (manufactured by Nippon Paper Industries Co., Ltd.)) and silica (Godd ball E-16C (manufactured by Suzukiyushi Industrial Corporation)). The tactile sensation of the obtained body powder was evaluated by the method described above. Table 11 shows the results.

### (Example B-15)

A solid powder eye shadow was prepared in the same manner as in Example B-8, except that mica (mica Y-2300X (manufactured by Yamaguchi Mica Co., Ltd.) in Table 10 was changed to a mixture containing the same weight of mica (mica Y-2300X (manufactured by Yamaguchi Mica Co., Ltd.)), synthetic mica (PDM-10L (manufactured by Topy Industries, Ltd.)), and (magnesium/potassium/silicon)/(fluoride/hydroxide/oxide) (micromica MK-200K (manufactured by Katakura & Co-op Agri Corporation)), and sericite was changed to a mixture containing the same weight of barium sulfate (plate-like barium sulfate H (manufactured by Sakai Chemical Industry Co., Ltd.)) and boron nitride (SHP-6 (manufactured by Mizushima Ferroalloy Co., Ltd.)). The tactile sensation of the obtained solid powder eye shadow was evaluated by the method described above. Table 11 shows the results.

### (Example B-16)

A liquid foundation was prepared in the same manner as in Example B-1, except that BG in Table 3 was changed to a mixture containing the same weight of glycerin and pentylene glycol (Diol PD (manufactured by Kokyu Alcohol Kogyo Co., Ltd.)). The tactile sensation of the obtained liquid foundation was evaluated by the method described above. Table 11 shows the results.

### (Example B-17)

A sunscreen was prepared in the same manner as in Example B-2, except that BG in Table 4 was changed to a mixture containing the same weight of glycerin and pentylene glycol (Diol PD (manufactured by Kokyu Alcohol Kogyo Co., Ltd.)). The tactile sensation of the obtained sunscreen was evaluated by the method described above. Table 11 shows the results.

### (Example B-18)

A makeup base was prepared in the same manner as in Example B-4, except that 1,3-butylene glycol in Table 6 was changed to a mixture containing the same weight of glycerin and pentylene glycol (Diol PD (manufactured by Kokyu Alcohol Kogyo Co., Ltd.)). The tactile sensation of the obtained makeup base was evaluated by the method described above. Table 11 shows the results.

### (Comparative Examples B-1 to B-8)

In Comparative Examples B-1 to B-8, a liquid foundation, a sunscreen, a powder foundation, a makeup base, a primer for lipsticks, a body powder, a solid white powder, and a solid powder eye shadow were prepared in the same manner as in Examples B-1 to B-8, respectively, except that the flattened particle (CA particle) of Example A-1 in Tables 3 to 10 was changed to the particle (CA particle) of Comparative Example A-1. The tactile sensation of each of them was evaluated in the method described above. Table 12 shows the results.

### [Table 11]

**(Table 11)**

| | Example B-1 | Example B-2 | Example B-3 | Example B-4 | Example B-5 | Example B-6 | Example B-7 | Example B-8 |
|---|---|---|---|---|---|---|---|---|
| Composition | Liquid Foundation | Sunscreen | Powder Foundation | Makeup Base | Primer for Lipsticks | Body Powder | Solid White Powder | Solid Powder Eye Shadow |

| Particle | Example A-1 | Example A-1 | Example A-1 | Example A-1 | Example A-1 | Example A-1 | Example A-1 | Example A-1 |
|---|---|---|---|---|---|---|---|---|
| Tactile Sensation | 4.5 | 4.6 | 4.3 | 4.2 | 4.1 | 4.5 | 4.7 | 4.6 |

| | Example B-9 | Example B-10 | Example B-11 | Example B-12 | Example B-13 | Example B-14 | Example B-15 | Example B-16 |
|---|---|---|---|---|---|---|---|---|
| Composition | Liquid Foundation | Sunscreen | Powder Foundation | Makeup Base | Powder Foundation | Body Powder | Solid Powder Eye Shadow | Liquid Foundation |

| Particle | Example A-1 | Example A-1 | Example A-1 | Example A-1 | Example A-1 | Example A-1 | Example A-1 | Example A-1 |
|---|---|---|---|---|---|---|---|---|
| Particle | Example A-1 | Example A-1 | | | | | | |
| Tactile Sensation | 4.2 | 4.3 | 4.2 | 4.1 | 4.2 | 4.4 | 4.2 | 4.3 |
| Tactile Sensation | 4.3 | 4.3 | | | | | | |

### [Table 12]

**(Table 12)**

| | Comp. Example B-1 | Comp. Example B-2 | Comp. Example B-3 | Comp. Example B-4 | Comp. Example B-5 | Comp. Example B-6 | Comp. Example B-7 | Comp. Example B-8 |
|---|---|---|---|---|---|---|---|---|
| Compositio n | Liquid Foundation | Sunscreen | Powder Foundation | Makeup Base | Primer for Lipsticks | Body Powder | Solid White Powder | Solid Powder Eye Shadow |

| Particle | Comp. Example A-1 | Comp. Example A-1 | Comp. Example A-1 | Comp. Example A-1 | Comp. Example A-1 | Comp. Example A-1 | Comp. Example A-1 | Comp. Example A-1 |
|---|---|---|---|---|---|---|---|---|
| Tactile Sensation | 3.1 | 2.9 | 3.2 | 3.1 | 3.3 | 3.2 | 3.2 | 3.1 |

As indicated in Tables 11 and 12, the tactile sensation of the cosmetic compositions of Examples B-1 to B-18, which contained the flattened particle of the present disclosure, were all excellent, with scores of 4.0 or more, and particularly exhibited silky tactile sensation. Furthermore, because all cosmetic compositions contained flattened particles that contained a biodegradable polymer as a major ingredient, excellent biodegradability can be expected.

## Claims

1. A biodegradable flattened particle comprising
a biodegradable polymer as a major ingredient and
having an oblateness L/T, which is a ratio of an average long diameter L (um) to an average thickness T (pm), of 2.0 or more and
a surface smoothness of 80% or more.

2. The biodegradable flattened particle according to claim 1, which has an aspect ratio L/S, which is a ratio of an average long diameter L (um) to an average short diameter S (µm), of 2.0 or more.

3. The biodegradable flattened particle according to claim 1 or 2, wherein the biodegradable polymer is selected from the group consisting of a polysaccharide, a polysaccharide ester, and an aliphatic polyester.

4. The biodegradable flattened particle according to claim 3, wherein the polysaccharide is one or two types of polysaccharides selected from cellulose and starch.

5. The biodegradable flattened particle according to claim 3, wherein the polysaccharide ester has a total substitution degree of over 0 and 3.0 or less.

6. The biodegradable flattened particle according to claim 3, wherein the polysaccharide ester is a cellulose acylate with a C2-10 acyl group.

7. The biodegradable flattened particle according to claim 3, wherein the aliphatic polyester is a polyhydroxyalkanoic acid or a polymer of an aliphatic dicarboxylic acid and an aliphatic diol.

8. The biodegradable flattened particle according to claim 3, wherein the aliphatic polyester is one or two or more types of aliphatic polyesters selected from the group of consisting of polycaprolactone, polyhydroxybutyric acid, and polylactic acid.

9. A cosmetic composition comprising the biodegradable flattened particle according to any one of claims 1 to 8.

10. A method for producing the biodegradable flattened particle of any one of claims 1 to 8, the method comprising:
mixing a biodegradable polymer, a plasticizer, and a water-soluble polymer to prepare a mixture;
melt-kneading the mixture at a temperature of 200°C or higher and 280°C or lower to prepare a kneaded mixture;
pressurizing the kneaded mixture at a temperature lower than a melting point of the water-soluble polymer; and
removing the water-soluble polymer from the pressurized kneaded mixture.

11. The method for producing the biodegradable flattened particle according to claim 10, wherein the kneaded mixture is pressurized at a temperature of 150°C or higher and 200°C or lower.

12. The method for producing the biodegradable flattened particle according to claim 10 or 11, wherein the kneaded mixture is pressurized at a pressure of 500 MPa or higher.
